# EUROPEAN PATENT APPLICATION

(11) **EP 4 635 562 A1**
(43) Date of publication of application: **22.10.2025**
(21) Application number: 23903007.5
(22) Date of filing: 19.07.2023
(51) Int. Cl.: A61N 5/10, A61B 6/04

(54) **BED FOR RADIOTHERAPY EQUIPMENT**

(30) Priority: 14.12.2022 JP 2022199538
(71) Applicant: HITACHI HIGH-TECH CORPORATION, Tokyo 105-6409 (JP)
(72) Inventor: SAITOH, Yu, Tokyo 100-8280 (JP); TSUMOTO, Yoshitaka, Tokyo 100-8280 (JP); ISHIZUKA, Takashi, Tokyo 100-8280 (JP); YOSHIDA, Koki, Tokyo 100-8280 (JP)
(74) Representative: MERH-IP Matias Erny Reichl Hoffmann Patentanwälte PartG mbB
(86) International application number: PCT/JP2023/026433
(87) International publication number: WO 2024/127701

(57) **Abstract**

Highly accurate inclination control of a bed for a radiotherapy device is implemented by a simple operation.

The bed for the radiotherapy device includes the top plate 6 as a bed configured to allow a radiotherapy subject to lie thereon, and the tilting unit 5 configured to change an inclination of the top plate 6. The tilting unit 5 includes a first bearing (the bearing 19) configured to pivotally support a first flat plate member (the lower plate 11) and a second flat plate member (the upper plate 12) while maintaining a predetermined distance therebetween, a first guide member (the linear guide 15) provided parallel to the first flat plate member, the movement unit 17 configured to move the first guide member, a second guide member (the linear guide 16) provided in the movement unit 17 and inclined with respect to the first flat plate member, the connection member 20 connecting a second bearing (the bearing unit 18) and the second flat plate member with a predetermined distance therebetween, the second bearing being configured to move the second guide member, and a biasing unit (the elastic body 23) configured to selectively bias the first flat plate member and the second flat plate member with the first bearing as a rotation axis to cause an angle formed by the first flat plate member and the second flat plate member to be in a specific direction.

## Description

### Technical Field

The present invention relates to a bed for a radiotherapy device.

### Background Art

In the related art, JP2012-070880A (PTL 1) describes a technique to adjust an inclination of a radiotherapy device. This publication states that "a couch drive device includes a first frame supported so as to be movable in parallel to the foundation, a second frame supported so as to be rotatable about a first rotation axis with respect to the first frame, a rotational movement drive device that moves a guide in parallel to a first direction with respect to the first frame, a slider supported by the guide so as to be movable in parallel to a second direction that is not parallel to the first direction, and a parallel movement drive device that moves the second frame in parallel to the foundation, and the slider is supported by the second frame so as to be rotatable about a second rotation axis different from the first rotation axis".

### Citation List

### Patent Literature

PTL 1: JP2012-070880A

### Summary of Invention

### Technical Problem

According to PTL 1, it is possible to control an inclination of a body axis of a treatment subject with respect to an O-ring of the radiotherapy device, that is, so-called pitch control.

However, in the related art, a decrease in accuracy due to tolerance is not considered. For example, when there is backlash due to a tolerance of a bearing, a position may not be reproduced when the sign of the rotation direction changes, and the positional accuracy required for treatment may not be satisfied.

In PTL 1, roll control, that is, control for rotating the treatment subject to the left and right while maintaining the inclination of the body axis of the treatment subject is not considered. For example, when two sets of mechanisms disclosed in PTL 1 are disposed in parallel and individually controlled, the roll and the pitch can be controlled simultaneously. However, in the mechanism that simultaneously controls the roll and the pitch on the same plane, the position changes depending on the order of adjustment of the roll and the pitch, and the operation becomes complicated.

An angle formed by the first direction and the second direction in PTL 1 affects the accuracy of pitch control, and therefore, it is required to improve the accuracy by reducing the angle formed by the first direction and the second direction.

Therefore, an object of the invention is to provide a bed for a radiotherapy device, which is capable of performing highly accurate inclination control with a simple operation.

### Solution to Problem

In order to achieve the above object, a typical bed for a radiotherapy device according to one aspect of the invention includes: a top plate as a bed configured to allow a radiotherapy subject to lie thereon; and a tilting unit configured to change an inclination of the top plate. The tilting unit includes a first bearing configured to pivotally support a first flat plate member and a second flat plate member while maintaining a predetermined distance therebetween, a first guide member provided parallel to the first flat plate member, a movement unit configured to move the first guide member, a second guide member provided in the movement unit and inclined with respect to the first flat plate member, a connection member connecting a second bearing and the second flat plate member with a predetermined distance therebetween, the second bearing being configured to move the second guide member, and a biasing unit configured to selectively bias the first flat plate member and the second flat plate member with the first bearing as a rotation axis to cause an angle formed by the first flat plate member and the second flat plate member to be in a specific direction.

### Advantageous Effects of Invention

The invention can provide a bed for a radiotherapy device, which is capable of performing highly accurate inclination control with a simple operation. Problems, configurations, and effects other than those described above will become apparent by the following description of embodiments.

### Brief Description of Drawings

FIG. 1 illustrates an example of an overall structure of a bed for a radiotherapy device.
FIG. 2 illustrates an example of a pitch-rotatable structure (part 1).
FIG. 3 illustrates an example of the pitch-rotatable structure (part 2).
FIG. 4 illustrates an example of the pitch-rotatable structure (part 3).
FIG. 5 illustrates an example of a roll-rotatable structure (part 1).
FIG. 6 illustrates an example of the roll-rotatable structure (part 2).
FIG. 7 illustrates an example of a structure rotatable about both a pitch axis and a roll axis.

### Description of Embodiments

Hereinafter, an embodiment will be described with reference to the drawings.

### [Embodiment 1]

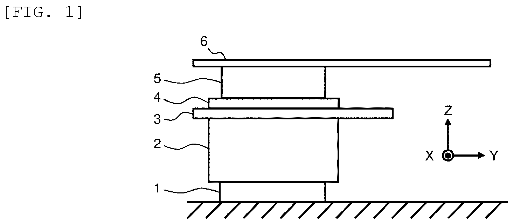

FIG. 1 illustrates an example of an overall structure of a bed for a radiotherapy device. A fixed unit 1 is a unit fixed to a floor surface, and a vertical movement unit 2 is a unit movable in a vertical direction (Z-axis direction) with respect to the floor surface. A left-right movement unit 3 is a unit that is movable in a horizontal direction (X-axis direction) with respect to the floor surface. A front-rear movement unit 4 is a unit that is movable in a direction perpendicular to the vertical movement unit 2 and the left-right movement unit 3 (horizontal direction with respect to the floor surface, Y-axis direction). A tilting unit 5 is a unit rotatable in a roll axis direction and a pitch axis direction. Here, the roll axis direction represents rotation about the Y axis, and the pitch axis direction represents rotation about the X axis. A top plate 6 is a portion on which a patient lies for treatment. The patient lies with a body axis parallel to the Y axis. Therefore, the pitch axis direction is a direction in which the inclination of the body axis is changed, and the roll axis direction is a direction in which the body of the patient is rotated while maintaining the inclination of the body axis. In FIG. 1, the fixed unit 1, the vertical movement unit 2, the left-right movement unit 3, the front-rear movement unit 4, the tilting unit 5, and the top plate 6 are disposed in this order from a floor surface side, but the invention is not limited thereto.

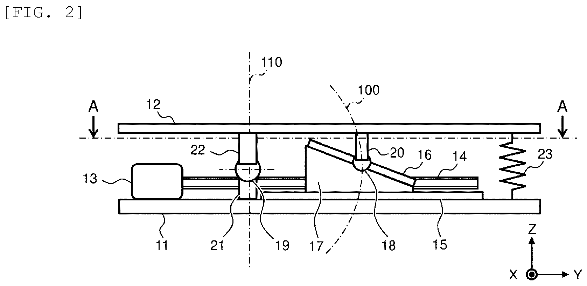

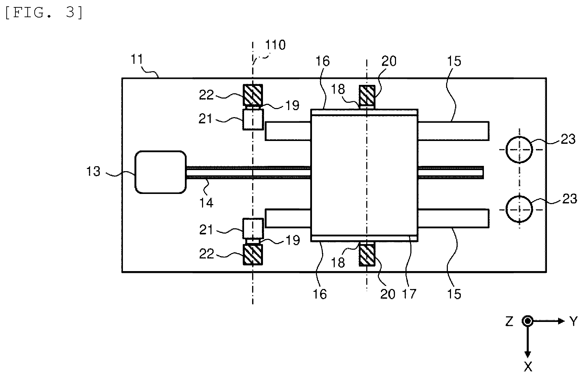

FIGS. 2 and 3 illustrate an example of a pitch-rotatable structure of the tilting unit 5. FIG. 3 illustrates a cross section taken along A-A in FIG. 2. A lower plate 11 is a first flat plate member, and an upper plate 12 is a second flat plate member. A motor 13 and a ball screw 14 coupled to the motor 13 are provided in a space between the lower plate 11 and the upper plate 12. A linear guide 15, which is a first guide member, is disposed on a surface parallel to the lower plate 11. A movement unit 17 is disposed on the linear guide 15 and is directly connected to the ball screw 14. Further, a linear guide 16, which is a second guide member, is disposed in the movement unit 17 in a direction inclined with respect to the lower plate 11, and a bearing 18 can travel along the linear guide 16. The bearing 18 is a second bearing. The bearing 18 is connected to the upper plate 12 via a connection member 20 rigidly fixed to the upper plate 12. A connection member 21 is rigidly fixed to the lower plate 11, and a connection member 22 is rigidly fixed to the upper plate 12. The connection members 21 and 22 are connected via a bearing 19. The bearing 19 is a first bearing. The lower plate 11 and the upper plate 12 are connected via an elastic body 23. A plurality of elastic bodies 23 may be provided, but it is necessary to dispose the elastic bodies 23 only on one side of a ZX plane 110 passing through an axis of the bearing 19, that is, a pitch rotation axis.

With the above structure, the upper plate 12 is rotatable in the pitch axis direction with the bearing 19 as a rotation center. That is, when the motor 13 rotates, the movement unit 17 moves in parallel along a direction of the linear guide 15. Then, the bearing 18 moves in parallel along the linear guide 16, and a rotation trajectory 100 is drawn with the bearing 19 as a center. Further, a rotation angle of the bearing 19 can be converted into a linear motion by the linear guide 15, so that highly accurate position control can be implemented.

Next, the role of the elastic body 23 will be described. For radiotherapy, accuracy on the order of 0.1 mm is required. On the other hand, the bearings 18 and 19 generally have backlash due to tolerance. When there is backlash, a position may not be reproduced when the sign of the rotation direction changes, and the positional accuracy required for treatment may not be satisfied. Deterioration in the positional accuracy due to the backlash can be prevented by appropriately setting the spring constant of the elastic body and continuously applying the elastic force to only one side (for example, the positive direction side of the rotation) of the backlash at all times.

In FIG. 3, two linear guides 15 are disposed in parallel along the Y axis, and the movement unit 17 is provided across the two linear guides 15. In this way, a roll angle can be easily and stably controlled by providing a plurality of linear guides 15 and bridging the movement unit 17.

The motor 13 and the ball screw 14 are located between the two linear guides 15. Therefore, for example, even if a length of the linear guide 15 is close to a length of the lower plate 11 in the Y direction, the linear guide 15 does not interfere with the motor 13 and the ball screw 14. When the linear guide 15 is lengthened, the difference in angle with the linear guide 16 can be reduced. This means that the operation amount of the motor can be increased with respect to the required control range of the pitch angle, which contributes to improvement in control accuracy.

When the linear guide 15 is lengthened, a notch may be provided in the movement unit 17 in order to avoid interference between the motor 13 and the movement unit 17. When the linear guide 15 is lengthened, the elastic body 23 may be disposed between the two linear guides, and a notch may be provided in the movement unit 17 in order to avoid interference between the elastic body 23 and the movement unit 17. For example, when the motor 13 and the elastic body 23 are located opposite to each other with respect to the movement unit 17 and a notch is provided to avoid interference with both, the movement unit 17 has an H shape.

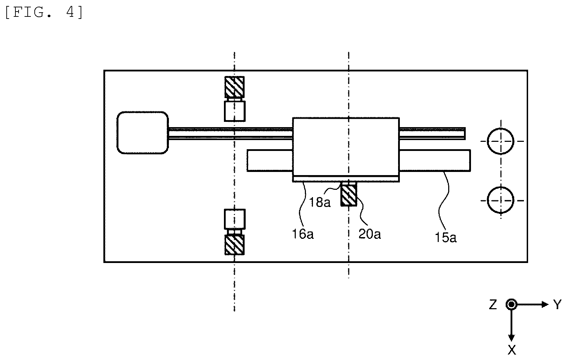

FIG. 4 illustrates another example of the cross section taken along A-A in FIG. 2. In this example, one linear guide 15a, one linear guide 16a, one bearing 18a, and one connection member 20a are used.

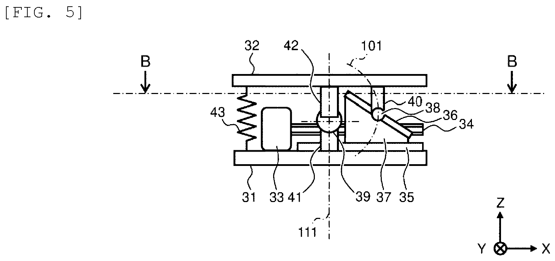

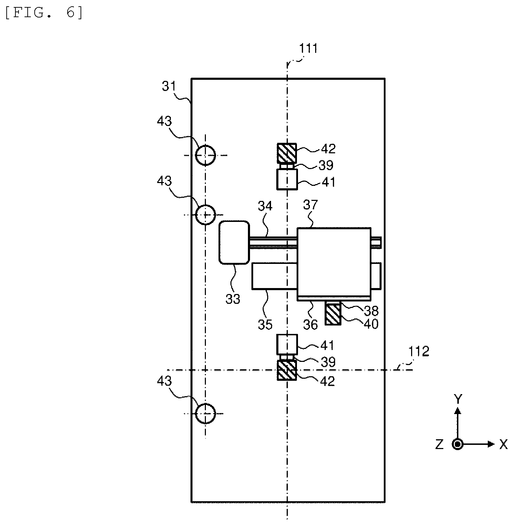

FIGS. 5 and 6 illustrate an example of a roll-rotatable structure of the tilting unit 5. FIG. 6 illustrates a cross section taken along B-B in FIG. 5. The structure is substantially the same as the pitch rotation structure (FIGS. 2 to 4) rotated by 90 degrees around the Z axis. Accordingly, when a motor 33 rotates, an upper plate 32 can rotate in a roll direction with a bearing 39 as a rotation center.

Specifically, a lower plate 31 is a first flat plate member, and the upper plate 32 is a second flat plate member. The motor 33 and a ball screw 34 coupled to the motor 33 are provided in a space between the lower plate 31 and the upper plate 32. A linear guide 35, which is the first guide member, is disposed on a surface parallel to the lower plate 11. A movement unit 37 is disposed on the linear guide 35 and is directly connected to the ball screw 34. Further, a linear guide 36, which is the second guide member, is disposed in the movement unit 37 in a direction inclined with respect to the lower plate 31, and a bearing 38 can travel along a linear guide 36. The bearing 38 is a second bearing. The bearing 38 is connected to the upper plate 32 via a connection member 40 rigidly fixed to the upper plate 32. A connection member 41 is rigidly fixed to the lower plate 31, and a connection member 42 is rigidly fixed to the upper plate 32. The connection members 41 and 42 are connected via a bearing 39. The bearing 39 is a first bearing. The lower plate 31 and the upper plate 32 are connected via an elastic body 43. A plurality of elastic bodies 43 may be provided, but it is necessary to dispose the elastic bodies 43 only on one side with respect to an axis of the bearing 39, that is, a ZY plane 111 passing through the roll rotation axis.

With the above structure, the upper plate 32 is rotatable in the roll axis direction with the bearing 39 as a rotation center. That is, when the motor 33 rotates, the movement unit 37 moves in parallel along a direction of the linear guide 35. Then, the bearing 38 moves in parallel along the linear guide 36, and a rotation trajectory 101 is drawn with the bearing 39 as a center. Further, a rotation angle of the bearing 39 can be converted into a linear motion by the linear guide 35, so that highly accurate position control can be implemented.

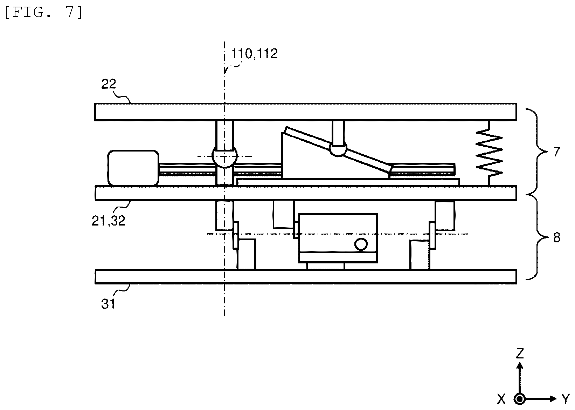

FIG. 7 illustrates an example of a structure of the tilting unit 5 that is rotatable about both the pitch axis and the roll axis. A pitch rotation structure 7 (FIGS. 2 to 4) and a roll rotation structure 8 (FIGS. 5 and 6) are stacked in the vertical direction. In this example, the lower plate 11 of the pitch rotation structure 7 and the upper plate 32 of the roll rotation structure 8 are common members.

The ZX plane 110 passing through the pitch rotation axis in the pitch rotation structure 7 and the ZX plane 112 passing through the connection member 42 in the roll rotation structure 8 are desirably the same plane. With such a structure, the pitch rotation structure 7 can smoothly transmit a force received from the top plate 6 to the roll rotation structure 8 side, which is advantageous in terms of strength.

In other words, when the support member of the bearing 19 in the other layer is located on a plane including the pitch rotation axis or the roll rotation axis and perpendicular to the floor surface, it is advantageous in terms of strength and contributes to improvement in accuracy of angle control.

As described above, when the layers for controlling the respective angles of the pitch rotation axis and the roll rotation axis which are perpendicular to each other are individually provided to form a stacked structure, the pitch and the roll can be independently controlled, and the state can be uniquely specified regardless of the order of control. It does not matter whether the layer for controlling the pitch and the layer for controlling the roll are placed above or below.

As described above, the disclosed bed for the radiotherapy device includes the top plate 6 as a bed configured to allow a radiotherapy subject to lie thereon, and the tilting unit 5 configured to change an inclination of the top plate **6.** The tilting unit 5 includes a first bearing (the bearing 19) configured to pivotally support a first flat plate member (the lower plate 11) and a second flat plate member (the upper plate 12) while maintaining a predetermined distance therebetween, a first guide member (the linear guide 15) provided parallel to the first flat plate member, the movement unit 17 configured to move the first guide member, a second guide member (the linear guide 16) provided in the movement unit 17 and inclined with respect to the first flat plate member, the connection member 20 connecting a second bearing (the bearing unit 18) and the second flat plate member with a predetermined distance therebetween, the second bearing being configured to move the second guide member, and a biasing unit (the elastic body 23) configured to selectively bias the first flat plate member and the second flat plate member with the first bearing as a rotation axis to cause an angle formed by the first flat plate member and the second flat plate member to be in a specific direction.

In this configuration, the biasing unit constantly biases, so that deterioration in positional accuracy due to backlash can be prevented, and highly accurate inclination control with a simple operation can be performed.

Further, the tilting unit 5 has a stacked structure obtained by stacking a lower layer (the roll rotation structure 8) close to a floor surface and an upper layer (the pitch rotation structure 7) far from the floor surface. Each of the lower layer and the upper layer includes the first flat plate member, the second flat plate member, the first bearing, the first guide member, the movement unit, the second guide member, the connection member, and the biasing unit. A lower rotation axis that is a rotation axis of the first bearing in the lower layer and an upper rotation axis that is a rotation axis of the first bearing in the upper layer are perpendicular to each other.

Therefore, the two rotation axes can be independently controlled, and the inclination can be controlled with high accuracy by a simple operation.

Further, the first flat plate member or the second flat plate member in the lower layer and the second flat plate member or the first flat plate member in the upper layer are the same flat plate member.

Therefore, the configuration can be simplified, and the cost can be reduced.

Further, a support member of the first bearing in the upper layer or the lower layer is located on a plane including the lower rotation axis or the upper rotation axis and perpendicular to the floor surface.

In this structure, a decrease in accuracy due to a load from the top plate can be prevented.

It is preferable that the biasing unit is an elastic body configured to bias the first flat plate member and the second flat plate member to increase the angle formed by the first flat plate member and the second flat plate member.

For example, the accuracy can be improved at low cost by using a coil spring or a plate spring.

Further, a plurality of the first guide members may be provided, and the movement unit may be provided across the plurality of first guide members.

In this configuration, the angle can be easily and stably controlled.

The invention is not limited to the above-described embodiment and includes various modifications. For example, the above-described embodiment has been described in detail to facilitate understanding of the invention, and the invention is not necessarily limited to those including all the configurations described above. In addition to deletion of such a configuration, it is also possible to replace or add a configuration.

As an example, a configuration in which the elastic body 23 as the biasing unit is disposed on only one side with respect to the axis of the bearing 19 has been described in the above-described embodiment, but a configuration in which the elastic bodies 23 are disposed on both sides of the bearing 19, a force is applied in a direction in which one extends, and a force is applied in a direction in which the other contracts may be adopted. Instead of the elastic body, a weight distribution may be used such that a force in a specific direction is always applied to the angle formed by the first flat plate member and the second flat plate member.

### Reference Signs List

- 1:: fixed unit
- 2:: vertical movement unit
- 3:: left-right movement unit
- 4:: front-rear movement unit
- 5:: tilting unit
- 6:: top plate
- 7:: pitch rotation structure
- 8:: roll rotation structure
- 11:: lower plate
- 12:: upper plate
- 13:: motor
- 14:: ball screw
- 15:: linear guide
- 16:: linear guide
- 17:: movement body
- 18:: bearing
- 19:: bearing
- 20:: connection member
- 21:: connection member
- 21:: lower plate
- 22:: upper plate
- 22:: connection member
- 23:: elastic body

## Claims

1. A bed for a radiotherapy device, the bed comprising:
a top plate as a bed configured to allow a radiotherapy subject to lie thereon; and
a tilting unit configured to change an inclination of the top plate, wherein
the tilting unit includes
a first bearing configured to pivotally support a first flat plate member and a second flat plate member while maintaining a predetermined distance therebetween,
a first guide member provided parallel to the first flat plate member,
a movement unit configured to move the first guide member,
a second guide member provided in the movement unit and inclined with respect to the first flat plate member,
a connection member connecting a second bearing and the second flat plate member with a predetermined distance therebetween, the second bearing being configured to move the second guide member, and
a biasing unit configured to selectively bias the first flat plate member and the second flat plate member with the first bearing as a rotation axis to cause an angle formed by the first flat plate member and the second flat plate member to be in a specific direction.

2. The bed for a radiotherapy device according to claim 1, wherein
the tilting unit has a stacked structure obtained by stacking a lower layer close to a floor surface and an upper layer far from the floor surface,
each of the lower layer and the upper layer includes the first flat plate member, the second flat plate member, the first bearing, the first guide member, the movement unit, the second guide member, the connection member, and the biasing unit, and
a lower rotation axis that is a rotation axis of the first bearing in the lower layer and an upper rotation axis that is a rotation axis of the first bearing in the upper layer are perpendicular to each other.

3. The bed for a radiotherapy device according to claim 2, wherein
the first flat plate member or the second flat plate member in the lower layer and the second flat plate member or the first flat plate member in the upper layer are the same flat plate member.

4. The bed for a radiotherapy device according to claim 2, wherein
a support member of the first bearing in the upper layer or the lower layer is located on a plane including the lower rotation axis or the upper rotation axis and perpendicular to the floor surface.

5. The bed for a radiotherapy device according to claim 1, wherein
the biasing unit is an elastic body configured to bias the first flat plate member and the second flat plate member to increase the angle formed by the first flat plate member and the second flat plate member.

6. The bed for a radiotherapy device according to claim 1, wherein
a plurality of the first guide members are provided, and
the movement unit is provided across the plurality of first guide members.
